# EUROPEAN PATENT SPECIFICATION

(11) **EP 4 486 265 B1**
(45) Date of publication and mention of the grant of the patent: **24.12.2025**
(21) Application number: 23710214.0
(22) Date of filing: 28.02.2023
(51) Int. Cl.: A61F 5/441, A61F 5/442, A61F 5/443, A61F 5/445

(54) **A COLLECTING BAG FOR AN OSTOMY APPLIANCE**
EIN AUFFANGBEUTEL FÜR EINE STOMAVORRICHTUNG
UN SAC COLLECTEUR POUR UN APPAREIL POUR STOMIE

(30) Priority: 03.03.2022 DK PA202270085
(43) Date of publication of application: 08.01.2025
(73) Proprietor: Coloplast A/S, 3050 Humlebaek (DK)
(72) Inventor: BECKER, Kim, 3400 Hilleroed (DK)
(86) International application number: PCT/DK2023/050033
(87) International publication number: WO 2023/165668

(56) References cited:
- EP-A1- 2 637 613
- EP-A1- 2 668 935
- EP-A2- 1 955 682
- WO-A1-2014/081889
- GB-A- 1 131 756
- US-A- 4 180 072
- US-B2- 8 273 065

## Description

A collecting bag for an ostomy appliance is provided.

### Background

Stomal output often contains body fluids and visceral contents that are aggressive to both the skin of a user and to ostomy appliances. In particular, these aggressive fluids have a detrimental effect on the efficiency and integrity of the adhesive materials that are applied to attach an ostomy appliance to the user's peristomal skin surface. Therefore, it is important for users of ostomy appliances that the peristomal skin surface is thoroughly cleaned before adhering a new appliance to the skin surface. If a fresh appliance is applied on an inadequately cleaned skin surface the adhesive material is likely being compromised and the adhesive material thus prone to prematurely fail. Experience have shown that the peristomal skin area can become badly soiled during wear of ostomy appliances. Further, associated foul smells from stomal wastes can be both highly unpleasant and even stigmatising to the user. The process of performing a thorough and adequate cleaning of the peristomal skin surface can be cumbersome and lengthy for users, often further being individuals suffering from reduced dexterity. Moreover, users typically need access at least to a water source and to tissues or toilet paper for the cleaning process. Particularly, if a change of the ostomy appliance becomes necessary when a user is in the public domain, the process of changing the appliance, and the requirements therefor, may become even more inconvenient and stress-inducing for the user.

Ostomists and health care professionals alike would welcome improvements in ostomy devices to avoid such inconvenience and stress.

Document EP 2 668 935 discloses the features of the preamble of claim 1.

### Summary

In one aspect, the present disclosure provides a collecting bag for an ostomy appliance. The collecting bag for an ostomy appliance is further defined by the appended claims.

### Brief Description of the Drawings

The accompanying drawings are included to provide a further understanding of embodiments and are incorporated in and constitute a part of this specification. The figures illustrate embodiments and together with the description explain principles of embodiments. Other embodiments and many of the intended advantages of all embodiments will be readily appreciated as they become better understood by reference to the following detailed description.

Various exemplary embodiments and details are described hereinafter, with reference to the figures when relevant. It should be noted that the figures may or may not be drawn to scale and that elements of similar structures or functions are represented by like reference numerals throughout the figures. It should also be noted that the figures are only intended to facilitate the description of the embodiments. They are not intended as an exhaustive description of the invention or as a limitation on the scope of the invention. In addition, an illustrated embodiment needs not have all the effects or advantages shown. An effect or an advantage described in conjunction with a particular embodiment is not necessarily limited to that embodiment and may be practiced in other embodiments even if not so illustrated, or if not so explicitly described.

The figures are listed as follows:
Figure 1 is a schematic cross-sectional sideview illustrating one embodiment of a collecting bag according to the invention.
Figure 2 is a schematic cross-sectional sideview illustrating one embodiment of a collecting bag according to the invention.
Figure 3 is a schematic plan view illustrating one embodiment of a collecting bag according to the invention.
Figure 4 is a schematic plan view similar to Fig. 3 and illustrating one embodiment of a collecting bag according to the invention.
Figure 5 is a schematic, perspective view illustrating an ostomy appliance including one embodiment of a collecting bag according to the invention.

### Detailed Description

In the following Detailed Description, reference is made to the accompanying drawings, which form a part hereof, and in which is shown by way of illustration specific embodiments in which the invention may be practiced. In this regard, directional terminology, such as "top," "bottom," "front," "back," "leading," "trailing," etc., is used with reference to the orientation of the Figure(s) being described. Because components of embodiments can be positioned in a number of different orientations, the directional terminology is used for purposes of illustration and is not necessarily limiting. It is to be understood that other embodiments may be utilized, and structural or logical changes may be made without departing from the scope of the present invention. The following detailed description, therefore, is not to be taken in a limiting sense, and the scope of the present invention is defined by the appended claims.

It is to be understood that individual features of the various exemplary embodiments described herein may be combined with each other, unless specifically noted otherwise.

In this disclosure, solid and liquid wastes from the bowels may be referred to as both "output," "waste(s)," and "fluids" interchangeably. A subject using the irrigation system may be referred to as "patient" or "user". However, in some cases, "user" can also relate or refer to a health care professional (HCP), such as a doctor/physician, nurse, or other person.

In this disclosure, 'axial direction' should generally be considered as vertical seen in relation to a top and bottom of the portable base. The radial direction should be understood as transverse to the axial direction. In some sentences, the words "inner" and "outer" can be used. These qualifiers should generally be perceived with reference to the radial direction, such that a reference to an "outer" element means that the element is farther away from a centre portion of a component than an element referenced as "inner". In addition, "innermost" should be interpreted as the portion of a component forming a centre of the component and/or being adjacent to the centre of the component. In analogy, "outermost" should be interpreted as a portion of a component forming an outer edge or outer contour of a component and/or being adjacent to that outer edge or outer contour. In further analogy this is also the case with regard to the use of the words "uppermost" and "lowermost".

The phrase "adjacent" should be interpreted as being "close to and/or in contact with, or capable of temporarily or permanently to be brought into contact with, but not attached to [something]."

The use of phrases such as (but not limited to) "substantially" and "approximately" as qualifiers of certain features or effects in this disclosure, is intended to simply mean that any deviations are within tolerances that would normally be expected by the skilled person in the relevant field.

The use of the word "generally" as a qualifier to certain features or effects in this disclosure is intended to mean - for a structural feature: that a majority or major portion of such feature exhibits the characteristic in question, and - for a functional feature or an effect: that a majority of outcomes involving the characteristic provide the effect, but that exceptionally some outcomes may not provide the effect.

In the present disclosure, "insert member" is to be understood as an element of the claimed collecting bag which is formed as an addition to the back and front walls of polymeric film commonly making up collecting bags and located between the back and front walls. However, the phrase should not be interpreted as meaning an element which the user of the collecting bag can or has to actively "insert" him- or herself. The insert member is integrated with the collecting bag at manufacture.

Embodiments of the present disclosure provide a collecting bag for an ostomy appliance including an insert member which includes two layers of polymeric film located between a proximal wall and a distal wall of the collecting bag at least at a top half of the bag. One of the layers of polymeric film of the insert member comprises a penetrable or weakened zone which is adapted to be penetrated by a conduit or similar by a means for delivering a cleaning liquid. Embodiments provide a collecting bag which makes it possible for the user to clean the peristomal skin surface or area with a cleaning liquid with the collecting bag still being attached to the user's body. This provides for the "old" collecting bag to remain "in place", connected to the user's skin surface, and to use the "old" bag to collect the cleaning liquid used for cleaning the peristomal skin surface before removing the "old" appliance (including the bag) and replacing it with a new appliance. Embodiments further provide a collecting bag that makes it possible for the user to inspect the peristomal skin surface, which is particularly useful during a process of cleaning the peristomal skin surface. Embodiments further provide a collecting bag wherein the gas volume from a sudden burst of flatulence from the stoma can be contained in the collecting bag, because the insert member is configured to be able to expand allowing for the bag to quickly increase the available gas collecting volume. Embodiments further provide a collecting bag that provides additional protection of a gas filter located inside the collecting bag.

Thus, in a first **aspect,** the present disclosure relates to a collecting bag for an ostomy appliance, comprising a first layer and a second layer of polymeric film attached to each other along at least a portion of a peripheral edge of the bag. The first layer of film provides a proximal wall of the collecting bag, and the second layer of film provides a distal wall of the collecting bag. An inlet opening is provided in the proximal wall. The inlet opening is an opening through which the stomal output can enter into a collecting volume of the bag. The collecting bag includes a gas exit provided in the distal wall. The gas exit allows for flatulence gas coming from the stoma to exit the collecting bag, which would otherwise relatively quickly be filled by the flatulence gas. Commonly, a collecting bag includes a gas filter adapted to neutralize any foul smells of the stomal output and flatulence gasses, e.g., by applying active carbon. In embodiments, a gas filter is incorporated into the collecting bag of the present disclosure. In embodiments, the gas filter and/or the gas exit can be provided on an internal surface of the collecting bag. In embodiments, the gas filter and/or the gas exit can be provided in top half of the distal wall. In embodiments, the gas filter and/or the gas exit can be provided on the distal wall at a top half of the collecting bag.

The collecting bag includes an insert member comprising a third layer and a fourth layer of polymeric film provided between the proximal wall and the distal wall of the collecting bag at least at a top half of the collecting bag. The top half of the bag can be understood as a portion of the bag extending from a top peripheral edge of the bag and approximately half the distance between the top peripheral edge and a bottom peripheral edge of the bag (in embodiments wherein the collecting bag has an outlet portion at its bottom, the extent of the outlet portion is not included in the determination of the distance). According to the disclosure, the third layer is attached to the first layer along a periphery of the first layer at least at the top half, and the fourth layer is attached to the second layer along a periphery of the second layer at least at the top half. A bottom edge portion of the third layer is connected to a bottom edge portion of the fourth layer.

According to the disclosure, the third layer and the fourth layer combine to provide the insert member. The insert member will be understood to form two further film layers between the proximal wall and the distal wall at least at the top half of the bag. It is further understood that the respective bottom edge portions of the third and fourth film layer are not attached or connected to the first and/or second film layers but are connected to each other. Thereby, it is understood that, when viewed in a cross-sectional side-view, the third and fourth film layers combine to form an insert member from film material the like of a V- or U-shape or form.

In embodiments, the bottom edge portion of the third layer and the bottom edge portion of the fourth layer connect to each other at a position below the position of the inlet opening in the proximal wall (when the bag is considered or viewed in a vertical orientation). In embodiments, the bottom edge portions of the third and fourth film layer are connected to each other at a location being closer to the bottom peripheral edge of the bag than to the top peripheral edge of the bag. One advantage of the insert member, which can be understood as providing the possibility for the collecting bag to expand (by extending or stretching the third and fourth polymeric film layers of the insert member), the available collecting volume of the bag can be increased.

Further, the collecting bag includes first and second complementary connection means. The complementary connection means facilitate shifting of the collecting bag between an unexpanded condition, in which the first and second connection means are connected, and an expanded condition in which the first and second connection means are disconnected from each other. The complementary connection means can include adhesive connections, push-button type connections, hook-and-loop type connections and other suitable connection means. In embodiments, first complementary connection means is/are provided on a distal surface of the third layer of polymeric film and second complementary connection means is/are provided on a proximal surface of the fourth layer of polymeric film. Thereby, the complementary connection means can be provided on, and face each other, on opposing (distal respectively proximal) surfaces of the third and fourth film layers of the insert member. When the first and the second connection means are disconnected from each other, the possibility of the collecting bag to expand by extending the insert member provides for excess gas volume from a sudden burst of flatulence from the stoma to be contained in the collecting bag without the risk of detachment of the adhesive from the user's body, which is an issue known in prior art ostomy appliances, being caused by such sudden ballooning. In the technical area of human wastes collecting bags "ballooning" is defined as a condition wherein the volume of gas in the collecting bag increases rapidly and exceeds a gas filter's flow capacity and therefore causes the collecting bag to inflate.

According to the disclosure, the third layer of polymeric film includes a penetrable zone. In embodiments, the penetrable zone can be configured as a weakened area or zone in the third layer of polymeric film. By penetration of the penetrable zone an access opening can be created in the third layer. Particularly, the penetrable zone can be adapted to be penetrated by a conduit or similar of a means for delivering a cleaning liquid. The conduit of the cleaning liquid means can thereby be pushed through the access opening in the third layer and a cleaning liquid can subsequently be ejected out of the conduit and onto the peristomal skin area through (via) the inlet opening in the proximal wall of the bag to clean the peristomal skin area. It is understood that the process of cleaning the peristomal skin area can be carried out when the insert member is in an expanded condition, in which the complementary connection means are disengaged from each other. By providing the penetrable zone and thereby the access opening for the cleaning liquid means in the third layer of the insert member, the access opening is protected or shielded from unintended entering of foreign bodies as well as from untimely entering of the cleaning liquid means. This is advantageous over collecting bags having an access opening in the distal wall of the bag. According to the present invention, the user has to actively decide to "open" the insert member to gain access to the penetrable zone and be capable of entering the cleaning liquid means therethrough. Furthermore, it is an advantage that the access opening also does not interfere with the possible provision of a comfort layer (e.g. a non-woven or textile material) provided to cover the external surface of the proximal and/or distal wall of the collecting bag.

In embodiments, the penetrable zone comprises a re-sealable aperture. Thereby, the penetrable zone can be made capable of closing again after removal of the cleaning liquid conduit, if necessary. Alternatively, or additionally, the re-sealable aperture can be configured to be manipulable by the user to temporarily provide an outlet for letting out an excess volume of (flatulence) gas to further avoid or reduce ballooning of the collecting bag. In embodiments, the penetrable zone includes at least a rubber material or composition. In embodiments, the rubber material is butyl rubber. Alternatively latex can be applied. In embodiments, the penetrable zone comprises a composition of butyl rubbers, fillers, and other constituents, e.g., but not limited to, emulgators and additives.

In embodiments, the penetrable zone comprises a layer of an aluminium foil. In embodiments, the penetrable zone includes a laminate of two or more layers of different materials. In one embodiment, the penetrable zone includes a laminate comprising an aluminium layer and at least one polymeric material layer. At least one of the layers of the laminate is substantially liquid and gas impermeable.

In embodiments, the penetrable zone is provided in the third layer of the insert member such that a centre of the penetrable zone generally aligns with a centre of the inlet opening in the proximal wall of the bag. This is particularly, but not exclusively, useful for making easy access for the user to clean the immediate peristomal area by facilitating the directing of the cleaning liquid through the inlet opening and immediately onto the soiled peristomal skin area without having to incur stresses on the first, second, third, and fourth polymeric films of the collecting bag.

In other embodiments, the penetrable zone can be generally offset from alignment (unaligned) with the inlet opening of the bag, e.g. (but not exclusively), the penetrable zone can be provided in the third layer closer to the top periphery of the collecting bag where the third layer is attached to the first layer. This location of the penetrable zone can help provide for ease of additional cleaning or rinsing of the internal surfaces of the proximal and distal walls of the collecting bag.

In embodiments, the third layer of film material includes a reinforcement provided around at least a portion of the penetrable zone. The reinforcement provides a certain stiffness or rigidity to the portion of the third layer around the penetrable zone which makes it easier for the user to control the process of penetrating the penetrable zone with a conduit of a cleaning liquid means. In embodiments, the reinforcement is provided as a thickened portion of the film material of the third layer. In embodiments, the reinforcement is provided as an additional material adhered or otherwise connected to the third layer at least in a portion around the penetrable zone. Other embodiments of suitable reinforcements can be acceptable.

In embodiments, a pulling strip is attached to the third layer of polymeric film material. The pulling strip is provided to facilitate easy handling of the third layer of film material, i.e. to make it easy to control the film material e.g. when a conduit of a cleaning liquid means is to be penetrated through the penetrable zone. In embodiments, the pulling strip is attached to the third layer of polymeric film at a position aligning with a centre axis through the inlet opening of the proximal wall of the collecting bag. In embodiments, the pulling strip includes a tab. In embodiments, the pulling strip includes an "eye" or other suitably formed through-going opening adapted to receive a finger of the user. In embodiments, suitable materials for the pulling strip include polymeric film or sheet materials or paper-based materials. In embodiments, the pulling strip is adhered to the third layer of polymeric film material. In embodiments, the pulling strip is heat laminated or welded to the third layer of polymeric film material.

In embodiments, the collecting bag includes an adhesive flange for attachment of the collecting bag to a skin surface around a stoma of a user. In embodiments, the adhesive flange is provided on an external surface of the proximal wall around the inlet opening. An external surface of the proximal wall is to be understood in relation to a use situation of the collecting bag such that the external surface is a surface of the collecting bag facing towards the exterior/surroundings of the bag and conversely, an internal surface of the proximal or distal wall would be facing towards the interior/the collecting volume of the bag. In embodiments, the adhesive flange can comprise a backing layer of a polymeric material. A skin adhesive is provided on one side of the backing layer. The skin adhesive can be covered by a release liner that protects the adhesive surface until use, where the release liner can be removed by the user. The other side of the backing layer, i.e., a side without skin adhesive on it, can be welded or heat laminated into attachment with the external surface of the proximal wall around the inlet opening.

In embodiments, the collecting bag includes a first coupling part for attachment of the collecting bag to an ostomy appliance base plate. A base plate of an ostomy appliance is typically in itself an adhesive flange configured to be attached to a skin surface around the stoma of a user. The base plate can be adapted to couple with a coupling part of a collecting bag. The coupling mechanism is typically provided either by means of an adhesive connection or by means of a mechanical connection. Thus, in embodiments the first coupling part of the collecting bag includes an adhesive flange. The adhesive flange of the first coupling part can be configured to couple to a receiving flange or second coupling half on a base plate of an ostomy appliance. In other embodiments, the first coupling part of the collecting bag includes a mechanical coupling part provided as a generally ring-shaped member of a polymeric, thermosetting material. The ring-shaped member of the first coupling part can be configured to couple to a receiving flange or second coupling half on a base plate of an ostomy appliance. In embodiments, the first coupling part is provided on an external surface of the proximal wall around the inlet opening.

In embodiments, the third layer of polymeric film is transparent. This provides for visual inspection of the stoma and the peristomal area surrounding the stoma through the third layer of the insert member. This is particularly useful in the situations where the user must clean the peristomal area by ejecting a cleaning liquid onto the peristomal area via a cleaning liquid conduit penetrating through the penetrable zone, as described above. In addition, the transparent third layer of film material is useful for the user to look through during attachment of the collecting bag around the stoma for increased precision in locating the collecting bag to the skin surface (e.g., ensuring optimal alignment of the stoma with the inlet opening in the collecting bag).

In embodiments, the fourth layer of polymeric film is transparent. This provides an option for the user to inspect particularly the distal wall and/or a bottom portion of the inside collecting volume of the bag. Moreover, a fourth layer of transparent polymeric film can allow for inspection of a gas filter provided on the internal surface of the second film layer making up the distal wall of the bag. In embodiments, both the third layer and the fourth layer of polymeric film is transparent. This provides for a large inspection "window" in the collecting bag and particularly for ease of observing a process of cleaning the peristomal area around the stoma. It further allows inspection of the collected contents of the bag.

In embodiments, a proximal surface of the fourth layer of polymeric film comprises a display of peristomal skin area cleaning instructions. Thereby, when the user is ready to change the used appliance for a new one, he or she can disengage the complementary connection means and expand insert member of the collecting bag, whereby it will be possible for the user to look down of the proximal surface of the fourth layer of polymeric film and follow the cleaning instructions provided on the display. In embodiments, the display includes one or more illustrations and/or pictograms. In embodiments, the display includes one or more words or text instructing the user on how to clean the peristomal area by injecting a cleaning liquid with a suitable means through the penetrable zone.

Overall, the collecting bag according to the disclosure provides improved support for a user to thoroughly clean the peristomal area using the collecting bag that is due for replacement to collect the discharged cleaning liquid and stomal wastes being cleaned away. This option is particularly helpful in that users can avoid lengthy preparations for the process of replacing a used ostomy appliance with a new one. User interviews have established that for the usual exchange of their ostomy appliance, many users use a regular kitchen bin bag and fix it to their trouser lining or belt below the stoma before removing the old appliance. Thereby, the kitchen bin bag can assist the user, partly in catching stomal output (should any output flow out of the stoma during the collecting bag exchange), but more pronounced as a handy way of quickly getting rid of the toilet or tissue paper the user is applying to clean the peristomal area in preparation for the attachment of a new ostomy appliance. Obviously, such a process is cumbersome and requires preparation and time, as well as requiring sufficient available physical space for the user to carry out the appliance exchange (which is often not easy to find if the user is in the public domain). With the collecting bag according to the present disclosure, the user has the option of maintaining the already used appliance in place on the body and clean the peristomal skin area with a cleaning liquid via a conduit or similar entered through the penetrable zone in the third layer of the insert member. In addition, in embodiments of the construction according to the disclosure, the user can inspect the collecting bag and the peristomal area and therefore also the cleaning thereof via the transparent third layer and/or fourth layer of polymeric film. Moreover, the injected cleaning liquid is collected in the used collecting bag and can be discarded therewith once the user is satisfied with the cleaning result and removes the used appliance and replaces it with a new one. In this regard, it is particularly advantageous that the insert member according to the disclosure provides for the collecting bag to expand and increase the collecting volume, such that is may also contain the used cleaning liquid and the stomal wastes being rinsed off the skin surface (in addition to already collected stomal wastes in the bag). Moreover, the provision of the insert member also offers an additional protection of a gas filter typically located on an internal surface of the distal wall of the collecting bag. Gas filters in(side) collecting bags are known to be exposed to clogging due to stomal wastes reaching the gas filter and obstructing the gas inlet. Thus, in embodiments, the insert member provides an additional physical barrier between the inlet opening of the collecting bag (where stomal wastes enter) and the location of the gas filter. Thus, it is understood that the collecting bag according to the disclosure provides advantages to the user both during normal use, during preparation for replacement of the collecting bag, and during application of a "new" ostomy appliance to the skin surface.

In embodiments wherein the ostomy appliance is a two-piece appliance, the option of cleaning the peristomal skin area using the old appliance bag to collect the cleaning liquid and waste is particularly useful, because it may help ensure a more regular and/or thorough cleaning of the skin area, in turn providing for the adhesive on the base plate of the two-piece appliance to be more protected from disintegration by aggressive stomal wastes.

In embodiments, the cleaning liquid means advantageously include a liquid container made from a polymeric material. The container can include a suitable lid or other opening for filling the container with a cleaning liquid, such as tap water or a saline solution. In embodiments, the polymeric material and the dimensions of the container are chosen to provide a relatively soft and/or pliable container which can be relatively easily squeezed by the user to press the cleaning liquid out or the container and onto the peristomal skin area through the access opening in the third layer. The container can advantageously be a bottle-type container. In embodiments, the cleaning liquid means include a conduit or pipe-like injector configured to engage with the container, e.g., forming an integral part of the lid, and further configured to be inserted through the penetrable zone of the insert member. An end portion of the injector includes a discharge opening or nozzle through which the cleaning liquid can be expelled onto the peristomal skin area of the user.

### Detailed description of the drawings

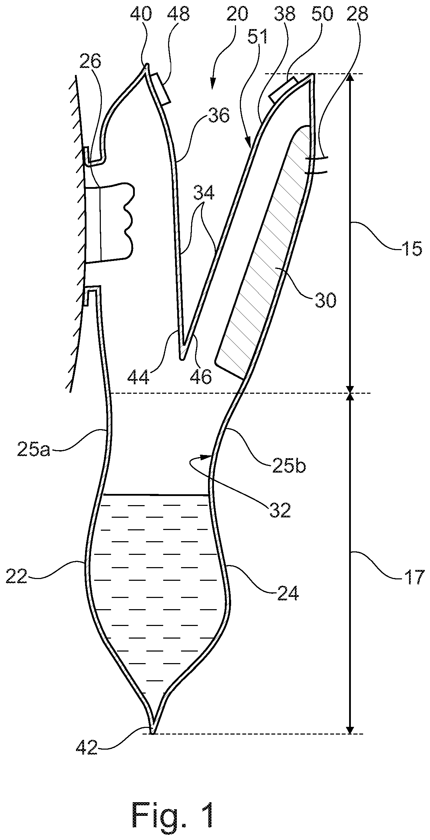

Figure 1 is a schematic cross-sectional sideview illustrating one embodiment of a collecting bag 20 according to the present invention. In Fig. 1, the collecting bag 20 is indicated to be attached to the skin area around a stoma of a user. The collecting bag 20 includes a first layer 22 and a second layer 24 of polymeric film. The first and second layers are attached to each other along at least a portion of a peripheral edge of the bag (not visible in Fig. 1). The first layer 24 of film forms a proximal wall 25a of the collecting bag 20, and the second layer of film 24 forms a distal wall 25b of the collecting bag 20. An inlet opening 26 is provided in the proximal wall 25a of the collecting bag, and the stoma of the user can be seen to protrude from the skin surface through the inlet opening 26 and into the collecting bag 20. In the embodiment of Fig. 1, the inlet opening 26 is provided in the proximal wall 25a in a location being at a top half 15 of the collecting bag 20. The collecting bag includes a gas exit 28 provided in the distal wall 25b. The gas exit 28 allows for flatulence gas coming from the stoma to exit the collecting bag 20. In the embodiment of Fig. 1, a gas filter 30 is provided on the internal surface 32 of the distal wall 25b of the collecting bag 20. In the embodiment of Fig. 1, the gas filter 30 and the gas exit 28 are provided on the distal wall 25b in the top half 15 of the collecting bag 20. The gas filter 30 filters foul smelling odours coming from the flatulence gas and from the collected stomal waste (indicated at the bottom / lower half 17 of the collecting bag 20).

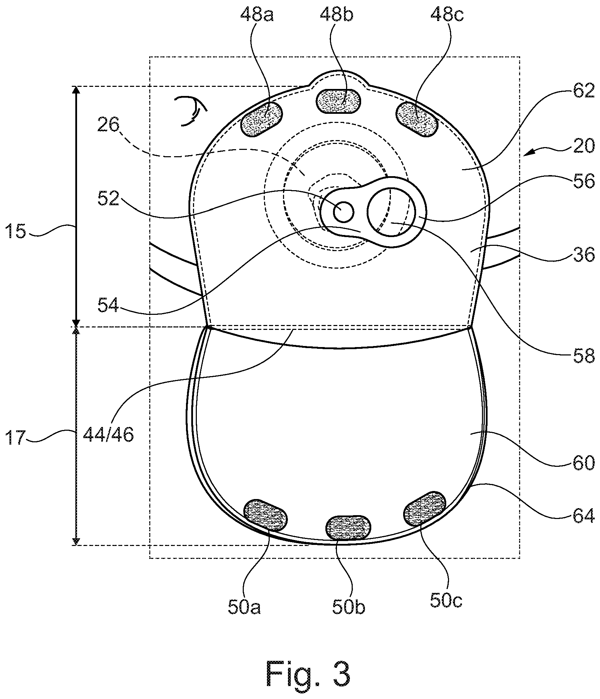

In the embodiment of Fig. 1, the collecting bag 20 includes an insert member 34 including a third layer 36 and a fourth layer 38 of polymeric film provided between the proximal wall 25a and the distal wall 25b of the collecting bag 20 at the top half 15 of the collecting bag 20. The top half 15 of the collecting bag 20 extends from a top peripheral edge 40 of the bag 20 and approximately half the distance between the top peripheral edge 40 and a bottom peripheral edge 42 of the bag. The third layer 36 is attached to the first layer 22 along a periphery of the first layer 22 at the top half 15, and the fourth layer 38 is attached to the second layer 24 along a periphery of the second layer 24 at the top half 15 (cf. Fig. 3). A bottom edge portion 44 of the third layer 36 of the insert member 34 is connected to a bottom edge portion 46 of the fourth layer 28 of the insert member 34.

As illustrated in Fig. 1, in embodiments the collecting bag 20 further includes first and second complementary connection means 48, 50. The complementary connection means 48, 50 facilitate shifting of the collecting bag 20 between an unexpanded condition, in which the first and second connection means 48, 50 are connected to each other, and an expanded condition in which the first and second connection means 48, 50 are disconnected from each other. In the embodiment of Fig. 1, the first and second connection means 48, 50 are disconnected, however the collecting bag 20 is not shown in the fully expanded condition with the insert member 34 being fully stretched. In the embodiment of Fig. 1, the complementary connection means 48, 50 include hook-and-loop type members. In the embodiment of Fig. 1, the first complementary connection means 48 is provided on a distal surface of the third layer 36 of polymeric film and the second complementary connection means 50 is provided on a proximal surface of the fourth layer 38 of polymeric film. Thus, in Fig. 1, the complementary connection means 48, 50 face each other on opposing surfaces of the third 36 and the fourth 38 film layers of the insert member 34.

In the Fig. 1 embodiment, a proximal surface of the fourth layer 38 of polymeric film comprises a display of cleaning instructions 51 describing (and/or illustrating) one or more possible procedures for cleaning of the peristomal skin area using the collecting bag 20 according to the invention. When the user has disengaged the complementary connection means 48, 50 and "opened" the insert member 34, it will be possible for the user to look down and consult the cleaning instructions provided on the display 51.

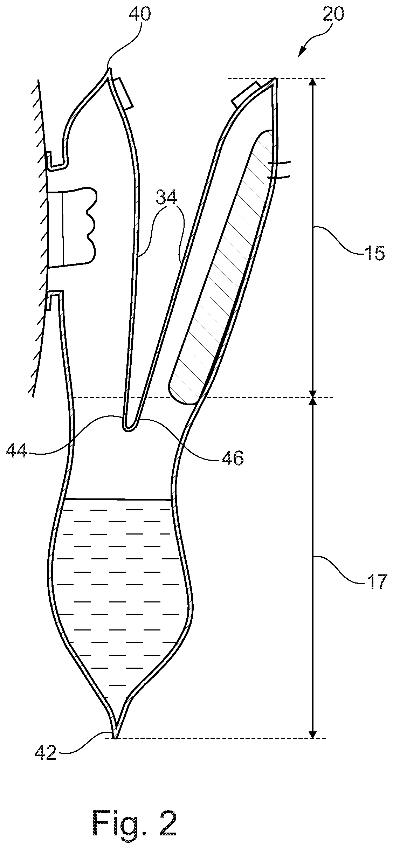

Figure 2 is a schematic cross-sectional sideview similar to Fig. 1 illustrating another embodiment of a collecting bag 20 according to the invention. In the embodiment of Fig. 2, the bottom edge portion 44 of the third layer 36 and the bottom edge portion 46 of the fourth layer 38 connect to each other at a location being closer to the bottom peripheral edge 42 of the bag 20 than to the top peripheral edge 40 of the bag 20. In the embodiment of Fig. 2, the greater extent of the insert member (in the vertical direction), provides for the available collecting volume of the bag 20 can be increased even further than in the embodiment of Fig. 1. In other words, the third layer 36 and the fourth layer 38 are connected to each other below the half-way point between the top half 15 and the bottom half 17 of the bag, indicated in Fig. 2 by a dotted line.

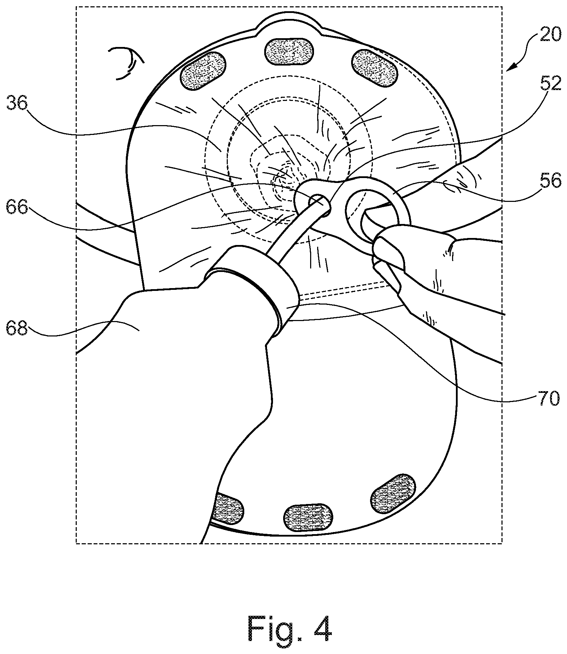

Figure 3 is a schematic plan view illustrating one embodiment of a collecting bag 20 according to the disclosure. Fig. 3 shows the collecting bag 20 in position around an artificial stoma attached to the stomach skin of a test person. In Fig. 3, the first and second complementary connection means 48, 50 are disconnected from each other and the insert member 34 is "opened". In Fig. 3, the first and second complementary connection means 48, 50 include three pairs (48a, 50a ; 48b, 50b ; 48c, 50c) of connections of the hook-and-loop type (e.g., a Velcro^{®} connection). In Fig. 3, the third layer 36 of polymeric film includes a penetrable zone 52. In the embodiment of Fig. 3, the penetrable zone 52 includes a weakened area in the third layer 36 of polymeric film. It is understood that the penetrable zone 52 is configured to effectively close off the collecting bag 20 until the penetrable zone 52 is penetrated by a cleaning liquid means (cf. Fig. 4). The weakened area of the penetrable zone 52 in Fig. 3 is configured as a plurality "kiss-cut" lines in the material of the penetrable zone 52, radiating from a common centre ("kiss-cutting" in the sense here being the process of cutting only partially through a material or laminate to provide a partial pre-perforation).

In the embodiment of Fig. 3, the penetrable zone 52 is provided in the third layer 36 of the insert member such that a centre of the penetrable zone 52 generally aligns with a centre of the inlet opening 26 in the proximal wall of the bag 20 (indicated by dotted line). This makes it easy to access and clean the immediate peristomal area by facilitating the directing of the cleaning liquid through the penetrable zone 52 and the inlet opening 26.

In the embodiment of Fig. 3, the third layer of film material 36 includes a reinforcement 54 provided around the penetrable zone 52. The reinforcement 54 provides a certain stiffness or rigidity to the portion of the third layer 36 around the penetrable zone 52 to make it easier for the user to penetrate the penetrable zone 52 with a conduit of a cleaning liquid means (cf. Fig. 4). In Fig. 3, the reinforcement 54 is provided as an additional material adhered to the third layer 36 around the penetrable zone 52.

In the embodiment of Fig. 3, a pulling strip 56 is attached to the third layer 36 of polymeric film material. Among other benefits, the pulling strip 56 makes it easier to handle the third layer film material 36 when the cleaning liquid means is to be penetrated through the penetrable zone 52. In Fig. 3, the pulling strip 56 includes an "eye" 58 adapted to receive a finger of the user. In Fig. 3, the pulling strip 56 includes a sheet material and forms part of the reinforcement 54 around the penetrable zone. In Fig. 3, the reinforcement 54/pulling strip 56 is adhered to the third layer 36 of polymeric film material around the penetrable zone 52, however it is understood that the portion of the pulling strip 56 including the eye 58 is not adhered to the third layer 36 but instead is freely accessible for the user.

In embodiments as in Fig. 3, the third layer 36 of polymeric film is transparent. This provides for visual inspection of the stoma and the peristomal area surrounding the stoma through the third layer 36 of the insert member and is particularly useful during cleaning. The fourth layer 38 of polymeric film material can also advantageously be transparent, however, as illustrated in Fig. 3, the fourth layer 38 can be covered (~overlaid) by e.g. a non-woven or other material layer 60, for example to improve ease of handling and/or to reinforce or strengthen the structure of the insert member 34.

In the embodiment of Fig. 3, it is illustrated how the third layer 36 is attached to the first layer 22 along a periphery 62 of the first layer 22 at the top half 15 of the bag 20. It can further be understood that the fourth layer 38 is attached to the second layer along a periphery 64 of the second layer at the top half 15. Note that in Fig. 3 the insert member is "opened" (the connection means 48, 50 are disconnected) and the fourth layer is therefore shown in a position where it is "tipped over" and located at (on top of in the schematic plan view of Fig. 3) the bottom half 17 of the bag 20. The bottom edge portion 44 of the third layer 36 of the insert member is connected to a bottom edge portion 46 of the fourth layer of the insert member.

Figure 4 is a schematic plan view similar to Fig. 3 and illustrating one embodiment of a collecting bag 20 according to the invention. In the embodiment of Fig. 4., the penetrable zone 52 is penetrated by a conduit 66 of a means for delivering a cleaning liquid in the form of liquid container 68. The conduit 66 of the container 66 of the cleaning liquid means is pushed through an access opening created in the penetrable zone 52 in the third layer 36 and a cleaning liquid can be squeezed out of the container 68 via the conduit 66 and onto the peristomal skin area to it. It is understood that the cleaning is carried out with the insert member in expanded condition as described above. In Fig. 4, the user has "opened" the insert member and gained access to the penetrable zone 52 while facilitating easy insertion of the cleaning liquid means 66, 68 by pulling on the pulling strip 56. The container 68 includes an openable lid 70 for filling the container with a cleaning liquid. In the embodiment of Fig. 4, the conduit 66 forms an integral part of the lid 70. The end portion of the conduit 66 includes a discharge opening or nozzle through which the cleaning liquid can be expelled onto the peristomal skin area of the user.

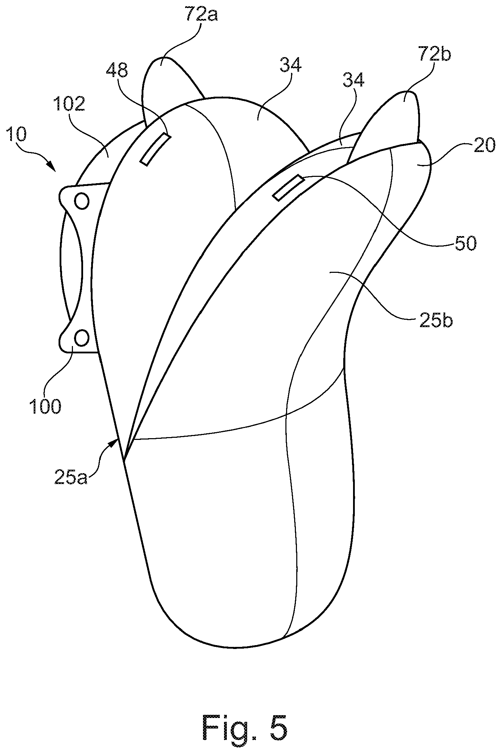

Figure 5 is a schematic, perspective view illustrating an ostomy appliance 10 including one embodiment of a collecting bag 20 according to the invention. In the embodiment of Fig. 5, the collecting bag 20 includes a first coupling part 100 for attachment of the collecting bag 20 to an ostomy appliance base plate 102, the base plate 102 in turn including an adhesive flange for attachment of the ostomy appliance to a skin surface around a stoma of a user. The base plate 102 comprises a second coupling part (not visible in Fig. 5) adapted to couple with the coupling part 100 of the collecting bag 20. In Fig. 5, the coupling mechanism is provided as a mechanical connection wherein the first coupling part 100 is a ring-shaped member of a polymeric, thermosetting material. The ring-shaped member of the first coupling part 100 is configured to couple to the second coupling half also being a ring-shaped member on the base plate 102 of an ostomy appliance. In the embodiment of Fig. 5, the first coupling 100 part is provided on an external surface of the proximal wall 25a around the inlet opening (not visible). Figure 5 illustrates the collecting bag 20 with the connection means 48, 50 disconnected from each other and the insert member 34 being in an "open" state. In Figure 5, the collecting bag 20 further includes gripping tabs 72a, 72b.

Although particular features have been shown and described, it will be understood that they are not intended to limit the claimed invention, and it will be made obvious to those skilled in the art that various changes and modifications may be made without departing from the scope of the claimed invention. The specification and drawings are, accordingly, to be regarded in an illustrative rather than restrictive sense.

## Claims

1. A collecting bag (20) for an ostomy appliance, comprising:
- a first layer (22) and a second layer (24) of polymeric film attached to each other along at least a portion of a peripheral edge of the bag, the first layer (22) of film providing a proximal wall (25a) of the collecting bag (20) and the second layer (24) of film providing a distal wall (25b) of the collecting bag (20);
- an inlet opening (26) provided in the proximal wall (25a);
- a gas exit (28) provided in the distal wall;
- an insert member (34) comprising a third layer (36) and a fourth layer (38) of polymeric film provided between the proximal wall (25a) and the distal wall (25b) at least at a top half of the bag (20), the third layer (36) attached to the first layer (24) along a periphery of the first layer (24) at least at the top half, and the fourth layer (38) attached to the second layer (24) along a periphery of the second layer (24) at least at the top portion, wherein a bottom edge portion of the third layer (36) is connected to a bottom edge portion of the fourth layer (38); and
- wherein the collecting bag (20) comprises first and second complementary connection means (48, 50) facilitating shifting of the collecting bag (20) between ar unexpanded condition, in which the first and second connection means (48, 50) are connected, and an expanded condition, in which the first and second connection means (48, 50) are disconnected from each other;
- **characterized in that** the third layer (36) of polymeric film comprises a penetrable zone (52).

2. The collecting bag (20) of claim 1, wherein the third layer (36) of polymeric film is transparent.

3. The collecting bag (20) of claim 1 or 2, wherein the fourth layer (38) of polymeric film is transparent.

4. The collecting bag (20) of any one of the preceding claims, further comprising ar adhesive flange provided on an external surface of the proximal wall (25a) around the inlet opening (26) for attachment of the collecting bag (20) to a skin surface around a stoma of a user.

5. The collecting bag (20) of any one of the preceding claims, wherein the bottom edge portion of the third layer (36) and the bottom edge portion of the fourth layer (38) connect to each other at a position below a position of the inlet opening (26) in the proximal wall (25a) when the collecting bag (20) is viewed in a vertical orientation.

6. The collecting bag (20) of any one of the preceding claims, wherein the penetrable zone (52) comprises a weakened area or zone in the third layer (36) of polymeric film.

7. The collecting bag (20) of any one of the preceding claims, wherein the penetrable zone (52) comprises a re-sealable aperture.

8. The collecting bag (20) of any one of the preceding claims, wherein the penetrable zone (52) is provided in the third layer (36) of the insert member such that a centre of the penetrable zone (52) generally aligns with a centre of the inlet opening (26) in the proximal wall (25a) of the bag (20).

9. The collecting bag (20) of any one of claims 1-7, wherein the penetrable zone (52) is generally offset from alignment with the inlet opening (26) in the proximal wall (25a) of the bag (20).

10. The collecting bag (20) of any one of the preceding claims, wherein the third layer (36) of film material comprises a reinforcement provided around at least a portion of the penetrable zone (52).

11. The collecting bag (20) of any one of the preceding claims, wherein a proximal surface of the fourth layer (38) of polymeric film comprises a display of cleaning instructions.

12. The collecting bag (20) of any one of the preceding claims, wherein a pulling strip (56) is attached to the third layer (36) of polymeric film material.

13. The collecting bag (20) of any one of the preceding claims, further comprising a gas filter (30).

14. The collecting bag (20) of any one of the preceding claims, wherein the bottom edge portion of the third layer (36) and the bottom edge portion of the fourth layer (38) connect to each other at a position being closer to the bottom peripheral edge of the bag (20) than to the top peripheral edge of the bag (20).

## Patentansprüche

1. Auffangbeutel (20) für eine Stomavorrichtung, umfassend:
- eine erste Schicht (22) und eine zweite Schicht (24) aus Polymerfolie, die zumindest entlang eines Teils eines Umfangsrands des Beutels aneinander befestigt sind, wobei die erste Schicht (22) der Folie eine proximale Wand (25a) des Auffangbeutels (20) bereitstellt und die zweite Schicht (24) der Folie eine distale Wand (25b) des Auffangbeutels (20) bereitstellt;
- eine Einlassöffnung (26), die in der proximalen Wand (25a) bereitgestellt ist;
- einen Gasauslass (28), der in der distalen Wand bereitgestellt ist;
- ein Einsatzelement (34), umfassend eine dritte Schicht (36) und eine vierte Schicht (38) aus Polymerfolie, die zwischen der proximalen Wand (25a) und der distalen Wand (25b) zumindest an einer oberen Hälfte des Beutels (20) bereitgestellt sind, wobei die dritte Schicht (36) an der ersten Schicht (24) entlang eines Umfangs der ersten Schicht (24) zumindest an der oberen Hälfte befestigt ist, und die vierte Schicht (38) an der zweiten Schicht (24) entlang eines Umfangs der zweiten Schicht (24) zumindest an dem oberen Abschnitt befestigt ist, wobei ein unterer Randabschnitt der dritten Schicht (36) mit einem unteren Randabschnitt der vierten Schicht (38) verbunden ist; und
- wobei der Auffangbeutel (20) erste und zweite komplementäre Verbindungsmittel (48, 50) umfasst, die ein Verschieben des Auffangbeutels (20) zwischen einem nicht expandierten Zustand, in dem die ersten und zweiten Verbindungsmittel (48, 50) verbunden sind, und einem expandierten Zustand, in dem die ersten und zweiten Verbindungsmittel (48, 50) voneinander getrennt sind, erleichtern;
**dadurch gekennzeichnet, dass** die dritte Schicht (36) aus Polymerfolie eine durchdringbare Zone (52) umfasst.

2. Auffangbeutel (20) nach Anspruch 1, wobei die dritte Schicht (36) aus Polymerfolie durchsichtig ist.

3. Auffangbeutel (20) nach Anspruch 1 oder 2, wobei die vierte Schicht (38) aus Polymerfolie durchsichtig ist.

4. Auffangbeutel (20) nach einem der vorhergehenden Ansprüche, ferner umfassend einen Klebeflansch, der auf einer Außenfläche der proximalen Wand (25a) um die Einlassöffnung (26) zum Befestigen des Auffangbeutels (20) an einer Hautoberfläche um ein Stoma eines Benutzers bereitgestellt ist.

5. Auffangbeutel (20) nach einem der vorhergehenden Ansprüche, wobei der untere Randabschnitt der dritten Schicht (36) und der untere Randabschnitt der vierten Schicht (38) an einer Position unterhalb einer Position der Einlassöffnung (26) in der proximalen Wand (25A) miteinander verbunden sind, wenn der Auffangbeutel (20) in einer vertikalen Ausrichtung betrachtet wird.

6. Auffangbeutel (20) nach einem der vorhergehenden Ansprüche, wobei die durchdringbare Zone (52) einen geschwächten Bereich oder eine Zone in der dritten Schicht (36) aus Polymerfolie umfasst.

7. Auffangbeutel (20) nach einem der vorhergehenden Ansprüche, wobei die durchdringbare Zone (52) eine wiederverschließbare Öffnung umfasst.

8. Auffangbeutel (20) nach einem der vorhergehenden Ansprüche, wobei die durchdringbare Zone (52) in der dritten Schicht (36) des Einsatzelements derart bereitgestellt ist, dass eine Mitte der durchdringbaren Zone (52) im Allgemeinen mit einer Mitte der Einlassöffnung (26) in der proximalen Wand (25a) des Beutels (20) ausgerichtet ist.

9. Auffangbeutel (20) nach einem der Ansprüche 1-7, wobei die durchdringbare Zone (52) im Allgemeinen von der Ausrichtung mit der Einlassöffnung (26) in der proximalen Wand (25a) des Beutels (20) versetzt ist.

10. Auffangbeutel (20) nach einem der vorhergehenden Ansprüche, wobei die dritte Schicht (36) aus Folienmaterial eine Verstärkung umfasst, die zumindest um einen Teil der durchdringbaren Zone (52) bereitgestellt ist.

11. Auffangbeutel (20) nach einem der vorhergehenden Ansprüche, wobei eine proximale Oberfläche der vierten Schicht (38) aus Polymerfolie eine Anzeige mit Reinigungsanweisungen umfasst.

12. Auffangbeutel (20) nach einem der vorhergehenden Ansprüche, wobei ein Zugstreifen (56) an der dritten Schicht (36) aus polymerem Filmmaterial befestigt ist.

13. Auffangbeutel (20) nach einem der vorhergehenden Ansprüche, ferner einen Gasfilter (30) umfassend.

14. Auffangbeutel (20) nach einem der vorhergehenden Ansprüche, wobei der untere Randabschnitt der dritten Schicht (36) und der untere Randabschnitt der vierten Schicht (38) an einer Position miteinander verbunden sind, die näher bei dem unteren Umfangsrand des Beutels (20) als bei dem oberen Umfangsrand des Beutels (20) ist.

## Revendications

1. Poche de collecte (20) pour un appareil de stomie, comprenant :
- une première couche (22) et une deuxième couche (24) de film polymère fixées l'une à l'autre le long d'au moins une partie d'un bord périphérique de la poche, la première couche (22) de film constituant une paroi proximale (25a) de la poche de collecte (20) et la deuxième couche (24) de film constituant une paroi distale (25b) de la poche de collecte (20) ;
- une ouverture d'entrée (26) ménagée dans la paroi proximale (25a) ;
- une sortie de gaz (28) ménagée dans la paroi distale ;
- un élément d'insertion (34) comprenant une troisième couche (36) et une quatrième couche (38) de film polymère disposées entre la paroi proximale (25a) et la paroi distale (25b) au moins au niveau d'une moitié supérieure de la poche (20), la troisième couche (36) étant fixée à la première couche (24) le long d'une périphérie de la première couche (24) au moins au niveau de la moitié supérieure, et la quatrième couche (38) étant fixée à la deuxième couche (24) le long d'une périphérie de la deuxième couche (24) au moins au niveau de la partie supérieure, une partie de bord inférieur de la troisième couche (36) étant reliée à une partie de bord inférieur de la quatrième couche (38) ; et
- dans lequel la poche de collecte (20) comprend des premier et second moyens de liaison complémentaires (48, 50) facilitant le décalage de la poche de collecte (20) entre un état non dilaté, dans lequel les premier et second moyens de liaison (48, 50) sont reliés, et un état dilaté, dans lequel les premier et second moyens de liaison (48, 50) sont désolidarisés l'un de l'autre ;
- **caractérisé en ce que** la troisième couche (36) de film polymère comporte une zone pénétrable (52).

2. Poche de collecte (20) selon la revendication 1, dans laquelle la troisième couche (36) de film polymère est transparente.

3. Poche de collecte (20) selon la revendication 1 ou 2, dans laquelle la quatrième couche (38) de film polymère est transparente.

4. Poche de collecte (20) selon l'une quelconque des revendications précédentes, comprenant en outre une bride adhésive fournie sur une surface externe de la paroi proximale (25a) autour de l'ouverture d'entrée (26) pour la fixation de la poche de collecte (20) à une surface de peau autour d'une stomie d'un utilisateur.

5. Poche de collecte (20) selon l'une quelconque des revendications précédentes, dans laquelle la partie de bord inférieur de la troisième couche (36) et la partie de bord inférieur de la quatrième couche (38) se raccordent l'une à l'autre au niveau d'une position au-dessous d'une position de l'ouverture d'entrée (26) dans la paroi proximale (25a) lorsque la poche de collecte (20) est vue dans une orientation verticale.

6. Poche de collecte (20) selon l'une quelconque des revendications précédentes, dans laquelle la zone pénétrable (52) comprend une surface ou zone affaiblie dans la troisième couche (36) de film polymère.

7. Poche de collecte (20) selon l'une quelconque des revendications précédentes, dans laquelle la zone pénétrable (52) comprend une ouverture refermable.

8. Poche de collecte (20) selon l'une quelconque des revendications précédentes, dans laquelle la zone pénétrable (52) est fournie dans la troisième couche (36) de l'élément d'insertion de telle sorte que le centre de la zone pénétrable (52) s'aligne généralement sur le centre de l'ouverture d'entrée (26) dans la paroi proximale (25a) de la poche (20).

9. Poche de collecte (20) selon l'une quelconque des revendications 1 à 7, dans laquelle la zone pénétrable (52) est généralement décalée de l'alignement sur l'ouverture d'entrée (26) dans la paroi proximale (25a) de la poche (20).

10. Poche de collecte (20) selon l'une quelconque des revendications précédentes, dans laquelle la troisième couche (36) de matériau en film comprend un renforcement prévu autour d'au moins une partie de la zone pénétrable (52).

11. Poche de collecte (20) selon l'une quelconque des revendications précédentes, dans laquelle une surface proximale de la quatrième couche (38) de film polymère comprend un affichage d'instructions de nettoyage.

12. Poche de collecte (20) selon l'une quelconque des revendications précédentes, dans laquelle une bande de traction (56) est fixée à la troisième couche (36) de matériau de film polymère.

13. Poche de collecte (20) selon l'une quelconque des revendications précédentes, comprenant en outre un filtre à gaz (30).

14. Poche de collecte (20) selon l'une quelconque des revendications précédentes, dans laquelle la partie de bord inférieur de la troisième couche (36) et la partie de bord inférieur de la quatrième couche (38) sont reliées l'une à l'autre au niveau d'une position qui est plus proche du bord périphérique inférieur de la poche (20) que du bord périphérique supérieur de la poche (20).
